# EUROPÄISCHE PATENTANMELDUNG

(11) **EP 0 565 030 A1**
(43) Veröffentlichungstag der Anmeldung: **13.10.1993**
(21) Anmeldenummer: 93105615.4
(22) Anmeldetag: 05.04.1993
(51) Int. Cl.: A62D 3/00, B01J 19/12

(54) **Vorrichtung zur Aufbereitung von Sondermüll**

(30) Priorität: 10.04.1992 DE 4212118
(71) Anmelder: MORTON INTERNATIONAL, INC., Chicago Illinois 60606-1596 (US)
(72) Erfinder: Horn, Klaus, Dr. Dipl.-Chem., W-6228 Hofheim (DE); Lingnau, Jürgen, Dr. Dipl.-Chem., W-6500 Mainz-Laubenheim (DE); Weiler, Horst, W-6229 Kiedrich (DE)
(74) Vertreter: Weber, Dieter, Dr.

(57) **Zusammenfassung**

Eine Vorrichtung 10 zur Aufbereitung von Sondermüll aus fotovernetzbarem Abfallmaterial 17 besteht aus einem Gehäuse 13, das auf einem Behälter 14 aufsitzt. Im Inneren des Gehäuses, das auf der Oberseite einen Eingabetrichter 6 aufweist, befinden sich ein oder mehrere UV-Strahler 1, die nahe dem Transportweg des Abfallmaterials 17 innerhalb des Gehäuses angeordnet sind. Diese UV-Strahler belichten das Abfallmaterial, so daß dieses polymerisiert wird. Unterhalb der UV-Strahler ist ein Schneidwerk 9 angebracht, in das das Abfallmaterial 17 eingeführt und durch Partikelschnitt zerkleinert wird. In dem durch eine Tür 18 zu öffnenden Behälter 14 befindet sich ein herausnehmbarer Abfallsack 19, in dem das von dem Schneidwerk 9 zerkleinerte Abfallmaterial hineinfällt.

## Beschreibung

Die Erfindung betrifft eine Vorrichtung zur Aufbereitung von Sondermüll aus fotovernetzbarem Abfall zu Hausmüll.

Fotovernetzbare Materialien, wie beispielsweise Trockenresiste, werden bei der Herstellung von Leiterplatten verwendet. Bei diesen Trockenresisten handelt es sich um lichtempfindliches Material in Folienform, das durch Laminieren mittels Druck und Wärme auf die Oberfläche eines Trägermaterials aufgebracht wird und durch Belichten und Entwickeln in eine Maskenschicht umgewandelt wird. Derartiges Material kann in der Regel nicht als Normalmüll, ähnlich dem Hausmüll, entsorgt werden, vielmehr handelt es sich um Sondermüll, der entsprechend aufwendig und damit kostenerhöhend entsorgt werden muß.

Darüber hinaus fallen beim Laminieren von Leiterplatten im allgemeinen Trockenresiststreifen als Abfallmaterial an, deren Oberflächen fotovernetzbar sind und die somit als Sondermüll entsorgt werden müssen.

Fotopolymerschichten, die durch Belichtung ihre Haftungseigenschaften ändern, werden beispielsweise bei der Herstellung von Farbprüffolien verwendet. Dabei handelt es sich meistens um lichtempfindliche Schichten, die sandwichartig zwischen zwei verschiedenen Trägermaterialien, im allgemeinen Folien, eingeschlossen sind, von denen eines zur Belichtung transparent ist. Erfolgt nach der Belichtung die Trennung der beiden Trägermaterialien, so bleiben die unbelichteten Teile der Fotopolymerschicht auf dem einen Trägermaterial haften, während die belichteten Teile der Fotopolymerschicht dagegen an dem anderen Trägermaterial haften.

Auch bei der Herstellung von solchen Farbprüffolien ergeben sich unbelichtete Abfallstreifen, d.h. fotovernetzbare Abfallstreifen, die als Sondermüll entsorgt werden müssen.

Ebenso fällt derartiger Sondermüll bei der Verarbeitung von Lötstoplack in Form von Soldermask an. Beim Lötstoplack handelt es sich dem Grunde nach gleichfalls um fotovernetzbaren Resist, dessen Verarbeitungsparameter und Belichtungsergebnisse etwas von denen für Trockenresist abweichen. Werden Trägermaterialien bzw. Druckfolien mit fotovernetzbarem Lötstoplack behandelt, so müssen die Abfälle gleichfalls als Sondermüll entsorgt werden.

Die in derartigen Systemen ebenfalls vorhandenen thermisch vernetzbaren Komponenten müssen durch Wärmeeinwirkung gleichfalls in nicht reaktives Material übergeführt werden. Ebenso können Abfälle von Siebdruckfolien durch Fotovernetzung als Normalmüll entsorgt werden.

Aufgabe der Erfindung ist es, eine Vorrichtung zu schaffen, die foto- und thermisch vernetzbare Abfälle aus Trockenresist, Lötstoplack, Farbprüffolien, Siebdruckfolien oder dergleichen mehr, die wegen ihrer reaktiven Bestandteile Sondermüll bilden, so aufbereitet, daß eine unmittelbare Entsorgung als Normal- bzw. Hausmüll möglich ist.

Zur Lösung dieser Aufgabe besteht die Vorrichtung zur Aufbereitung von Sondermüll aus fotopolymerisierbarem Abfall zu Hausmüll aus einem mit einem Eingabetrichter ausgerüsteten Gehäuse, in dem ein oder mehrere UV-Strahler nahe dem Transportweg des Abfallmaterials angeordnet sind und das Abfallmaterial belichten und erwärmen und in dem ein Schneidwerk das Abfallmaterial durch Partikelschnitt zerkleinert.

In Ausgestaltung der Erfindung sitzt das Gehäuse als abnehm- oder abklappbarer Aufsatz auf einem, nach oben hin offenen, prismatischen Behälter auf, der eine Tür aufweist und einen herausnehmbaren Abfallsack für das zerkleinerte Abfallmaterial enthält.

Die weitere Ausgestaltung der Erfindung ergibt sich aus den Merkmalen der Unteransprüche.

Die verschiedenen Ausführungsformen der Vorrichtung können so gestaltet sein, daß das Schneidwerk dem oder den UV-Strahlern nachgeschaltet oder vorgeschaltet ist. Ebenso ist es möglich, daß in dem Gehäuse ein horizontales, über zwei Umlenkrollen endlos umlaufendes Transportband angeordnet ist, ein Eingabetrichter sich in das Innere des Gehäuses erstreckt und oberhalb des Transportbandes endet, zumindest ein UV-Strahler oberhalb des Transportbandes und im Abstand von dem Eingabetrichter angebracht ist und ein Schneidwerk seitlich versetzt zu dem und unterhalb des Transportbandes vorhanden ist.

Durch das Belichten und Erwärmen der fotovernetzbaren bzw. thermisch vernetzbaren Abfälle mit Hilfe des oder der UV-Strahler sowie durch das Zerkleinern der Abfälle, wobei entweder zuerst belichtet und danach zerkleinert oder zuerst zerkleinert und dann belichtet werden kann, werden die Abfälle durchpolymerisiert, so daß keine fotochemischen oder thermischen Prozesse mehr unter Einwirkung von Sonnenlicht ablaufen können bzw. Auslaugen durch Regen oder Sickerwasser nicht stattfinden kann. Daher kann eine Entsorgung derartig behandelter Abfälle als Hausmüll unbedenklich vorgenommen werden.

Mit der Vorrichtung nach der Erfindung wird somit der Vorteil erzielt, daß durch die Entsorgung der Abfälle als Hausmüll die Kosten für eine Entsorgung als Sondermüll eingespart werden können und daß darüber hinaus noch durch die Zerkleinerung der Abfälle in kleine Teilstreifen von etwa 10 bis 15 mm Länge, im Vergleich zu nicht zerkleinertem Abfallmaterial, eine erhebliche Volumeneinsparung erreicht wird.

Die Erfindung wird im folgenden anhand von zeichnerisch dargestellten Ausführungsbeispielen näher erläutert. Es zeigen:
- Figur 1: in teilweise aufgebrochener perspektivischer Darstellung eine erste Ausführungsform der Vorrichtung nach der Erfindung,
- Figur 2: in teilweise aufgebrochener perspektivischer Darstellung eine zweite Ausführungsform der Vorrichtung nach der Erfindung, und
- Figur 3: in schematischer Schnittansicht eine dritte Ausführungsform der Vorrichtung nach der Erfindung.

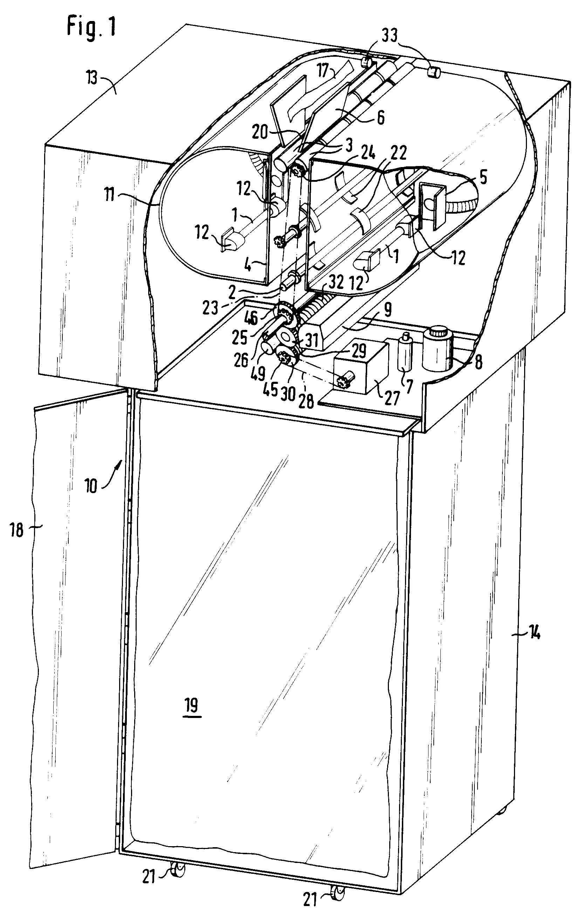

In Figur 1 ist eine erste Ausführungsform einer Vorrichtung 10 nach der Erfindung dargestellt. Die Vorrichtung 10 umfaßt ein Gehäuse 13, das auf einem prismatischen Behälter 14 aufsitzt. Das Gehäuse 13 ist entweder abnehmbar von dem Behälter 14 ausgebildet oder von diesem mittels eines nicht gezeigten Scharniers abklappbar, das sich beispielsweise über eine der Schmalseiten des Behälters 14 erstreckt. In dem Behälter 14, der durch eine aufklappbare Tür 18 verschlossen ist, befindet sich ein herausnehmbarer Abfallsack 19, der das aus einem Schneidwerk 9 im Gehäuse 13 austretende zerkleinerte Abfallmaterial aufnimmt. In dem Gehäuse 13 befinden sich zu beiden Seiten des Transportweges des Abfallmaterials 17 UV-Strahler 1, die das Abfallmaterial belichten. In Figur 1 sind die UV-Strahler 1 in Halterungen 12 waagerecht gelagert. Obgleich dies in Figur 1 nicht dargestellt ist, können die UV-Strahler auch senkrecht in solchen Halterungen angeordnet sein. Den UV-Strahlern 1, die von gekrümmten UF-Reflektoren 11 umgeben sind, ist das Schneidwerk 9 in Transportrichtung des Abfallmaterials 17 nachgeordnet. Bei diesem Schneidwerk 9 handelt es sich um eine Einrichtung ähnlich einem Schredderwerk, wie sie beispielsweise in an und für sich bekannten Reißwolfeinrichtungen verwendet wird. Auf der Oberseite des Gehäuses 13 befindet sich ein Eingabetrichter 6, der in einen Eingabeschlitz 20 eines Einsatzes 4 übergeht. Im Eingabeschlitz 20 sind Einzugswalzen 3 für das Abfallmaterial 17 angeordnet, das in Figur 1 als Abfallstreifen schematisch angedeutet ist. Im Eingabeschlitz 20 befindet sich desweiteren eine Fotozelle 33, die das Ein- und Ausschalten eines Motors 27 steuert, der sowohl das Schneidwerk 9 als auch die Einzugswalzen 3 in Bewegung versetzt, wie nachstehend noch näher beschrieben werden wird. Die Fotozelle 33 steuert das Ein- und Ausschalten des Motors 27 entsprechend dem Ein- und Durchlauf des Abfallmaterials 17, das mit Hilfe von Flügeln 22, die auf Transportwellen 2 aufsitzen, in das darunter liegende Schneidwerk 9 befördert wird. Die Einzugswalzen 3, die Transportwellen 2 und das Schneidwerk 9 sind in dem Einsatz 4, der aus UV-durchlässigem Glas oder Kunststoff besteht, angeordnet. In Figur 1 sind die UV-Strahler 1 horizontal ausgerichtet, jedoch ist es ebenso möglich, daß die UV-Strahler senkrecht und entsprechend auch die dazugehörigen UV-Reflektoren 11 senkrecht angeordnet sind. Jeder der UV-Strahler 1 wird mit Kühlluft aus einer Luftzuführung 5 beaufschlagt, um die während des Strahlerbetriebs stark erwärmte Umgebungsluft des Strahlers abzukühlen. Die erwärmte Umgebungsluft bewirkt beispielsweise die thermische Vernetzung von Abfällen, die neben einer fotovernetzbaren Komponente auch eine thermisch vernetzbare Komponente enthalten.

Wie schon erwähnt, erfolgt der Antrieb der Einzugswalzen 3 und des Schneidwerks 9 durch den Motor 27. Hierzu versetzt der Motor 27 über eine Zahnradkette 28 eine erste Schneidwelle 29 des Schneidwerks 9 in Bewegung, wobei die Zahnradkette 28 über ein Zahnrad 45 geführt ist, das als Stirnzahnrad auf der ersten Schneidwelle 29 des Schneidwerks 9 aufsitzt. Desweiteren läuft die Zahnradkette 28 über ein nicht näher bezeichnetes Stirnzahnrad, das auf der Antriebswelle des Motors 27 befestigt ist. Auf der ersten Schneidwelle sitzt desweiteren ein Zahnrad 30 auf, das unmittelbar an das Zahnrad 45 angrenzt. Das Zahnrad 30 kämmt mit einem Zahnrad 31 auf einer zweiten Schneidwelle 32 des Schneidwerks 9. Dieses Zahnrad 31 wiederum ist mit einem weiteren Zahnrad 46 auf der Welle 26 des Schneidwerks 9 im Eingriff. Das Zahnrad 46 grenzt an das Zahnrad 25 an. Über das Zahnrad 25 und ein Zahnrad 24 auf der Achse der einen Einzugswalze 3 ist eine endlos geschlossene Zahnradkette 23 geführt. Der Motor 27 treibt somit über die Zahnradkette 28, die Zahnräder 30, 31 und 46 sowie die Zahnradkette 23 gleichzeitig das Schneidwerk 9 und die Einzugswalzen 3 an.

Für den Einschaltvorgang der UV-Strahler 1 zur Begrenzung der Stromaufnahme sind eine Drossel 8 und ein Starter 7 vorgesehen. Die UV-Strahler 1 sind durch einen nicht gezeigten Schalter einschaltbar, der beim Aufsitzen des Gehäuses 13 auf dem Behälter 14 betätigt wird. Sobald das Gehäuse 13 abgehoben bzw. von dem Behälter 14 abgeklappt wird, wird die Stromzufuhr zu den UV-Strahlern durch den Schalter unterbrochen. Die Fotozelle 33 steuert, wie schon voranstehend erwähnt wurde, Start und Stop der Einzugswalzen und des Schneidwerks, und nach dem Durchlauf des Abfallmaterials 17 durch den Strahl der Fotozelle 33 erfolgt mit einer gewissen Verzögerung die automatische Abschaltung sowohl der Einzugswalzen, des Schneidwerks als auch der UV-Strahler.

Beim Zerkleinern des Abfalls im Schneidwerk 9 kommt es zu starken elektrostatischen Aufladungen der entstehenden Flitterpartikel, was dazu führt, daß diese Flitterpartikel an verschiedenen Stellen der Vorrichtung im Inneren des Gehäuses 13 haften. Um dies zu verhindern, ist eine Ionisiereinrichtung 49, beispielsweise ein mit hochfrequenter Spannung beaufschlagter Ionisierstab, im Gehäuseinneren in Nähe des Schneidwerks 9, im allgemeinen unterhalb der Schneidwellen, angebracht. Durch diese Ionisiereinrichtung wird die Umgebungsluft des Schneidwerks ionisiert und dadurch die elektrostatischen Ladungen von den Flitterpartikeln abgeleitet, so daß diese nicht mehr länger an den verschiedenen Stellen im Geräteinneren haften.

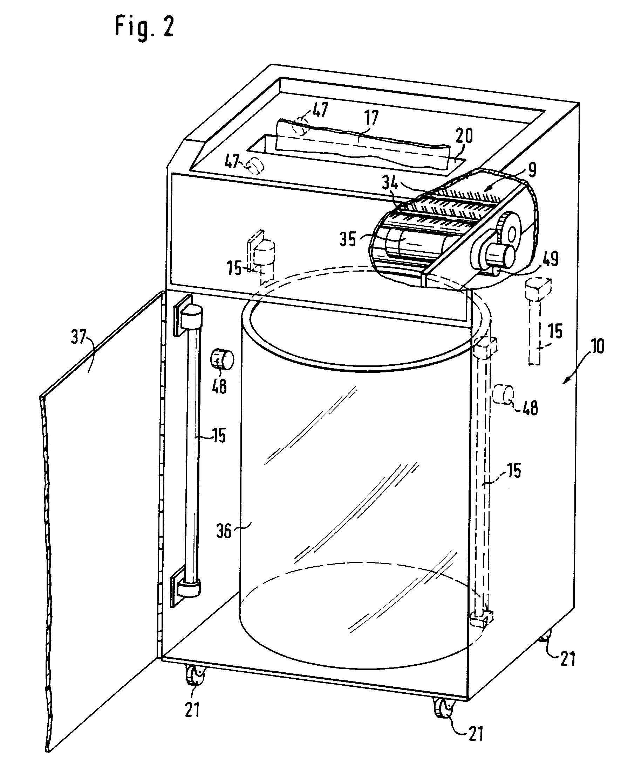

Figur 2 zeigt eine weitere Ausführungsform der Vorrichtung 10 nach der Erfindung. Bei dieser Ausführungsform ist das Schneidwerk 9 den UV-Strahlern vorgeschaltet und befindet sich unterhalb des Eingabeschlitzes 20. Das Schneidwerk 9 führt einen Partikelschnitt aus, wobei die Partikelgröße im Bereich von 2 x (10-15) mm² liegt. Das Schneidwerk weist zwei Schneidwellen 34 auf, von denen eine direkt von einem Motor 35 angetrieben ist. Unterhalb des Schneidwerks 9 befinden sich eine Ionisiereinrichtung 49 und ein zylindrischer Einsatz 36, in den die Partikel des zerschnittenen Abfallmaterials 17 hineinfallen. Der Einsatz 36 besteht aus einem zylindrischen UV-durchlässigen Glas- oder Kunststoffbehälter, der nach Öffnen einer Klapptür 37 herausnehmbar ist. Um den Mantel des Einsatzes 36 sind außen in gleichen Abständen mehrere stabförmige UV-Strahler 15 senkrecht angeordnet, die bei verschlossener Klapptür 37 eingeschaltet sind und beim Öffnen dieser Klapptür abgeschaltet werden. Die UV-Strahler 15 können auch waagerecht an den Innenseiten des Gehäuses der Vorrichtung 10 angebracht sein. Ebenso ist es möglich, daß nur eine einzige UV-Strahlungsquelle entsprechender Stärke verwendet wird, unter der Voraussetzung, daß deren Strahlenstärke ausreicht, die Partikel des Abfallmaterials durchzupolymerisieren. Anstelle von stabförmigen UV-Strahlern können auch glühlampenähnliche UV-Strahlungsquellen eingesetzt werden.

Auch bei dieser zweiten Ausführungsform der Vorrichtung 10 ist, ähnlich wie bei der ersten Ausführungsform der Vorrichtung nach Figur 1, im Einzugsbereich, nämlich unterhalb des Eingabeschlitzes 20, eine Fotozelle 47 vorhanden, die den Start und Stop des Schneidwerks 9 und das Ein- und Ausschalten der UV-Strahler 15 nach dem Durchlauf des Abfallmaterials 17 durch den Eingabeschlitz 20 mit einer vorgegebenen Verzögerung steuert. Nach dem Durchlauf durch den Eingabeschlitz 20 erfolgt dann mit dieser vorgegebenen Verzögerung eine automatische Abschaltung der Vorrichtung 10. Desweiteren ist bei dieser Ausführungsform eine Füllstandsanzeige 48 vorhanden, die die Füllstandshöhe in dem zylindrischen Einsatz 36 anzeigt. Sobald der Einsatz 36 gefüllt ist, erfolgt ebenfalls eine automatische Abschaltung des Schneidwerks 9 und der UV-Strahler 15. Ein nicht dargestellter Sicherheitsschalter schaltet beim Öffnen der Klapptür 37, wenn der Einsatz 36 zur Entleerung herausgenommen werden soll, gleichfalls die Vorrichtung 10 ab.

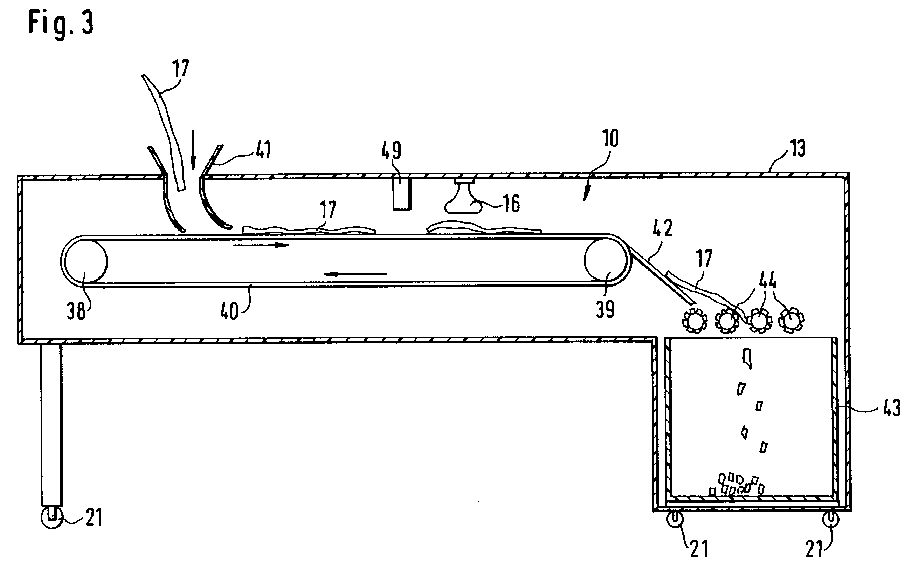

In Figur 3 ist schematisch eine dritte Ausführungsform der Vorrichtung 10 gezeigt, bei der in dem Gehäuse 13 ein horizontal, über zwei Umlenkrollen 38, 39 endlos umlaufendes Transportband 40 angeordnet ist. Durch einen Eingabetrichter 41 auf der Oberseite des Gehäuses 13 der Vorrichtung 10 wird das zu entsorgende Abfallmaterial 17 eingegeben. Der Eingabetrichter 41 erstreckt sich in das Innere des Gehäuses 13 und endet knapp oberhalb des Transportbandes 40. Dabei ist der Eingabetrichter 41 im Gehäuseinneren in Bewegungsrichtung des Obertrums des Transportbandes 40 gekrümmt. Das Abfallmaterial 17 wird in den Eingabetrichter 41 so eingeführt, daß die fotovernetzbare Schicht des Abfallmaterials beim Aufliegen auf dem Obertrum des Transportbandes 40 nach oben zeigt. Im Abstand von dem Eingabetrichter 41 sind eine Ionisiereinrichtung 49 und zumindest ein UV-Strahler 16 oberhalb des Transportbandes 40 angeordnet. An dem Transportband 40 liegt im Bereich der einen Umlenkrolle 39 ein Ableitelement 42 tangential an und lenkt das auf dem Transportband 40 aufliegende Abfallmaterial 17 in ein Schneidwerk 44 ab, das seitlich versetzt zu dem Transportband und unterhalb von diesem angeordnet ist. Dieses Schneidwerk 44 entspricht weitgehend dem Schneidwerk 9 nach den Figuren 1 und 2, so daß auf Einzelheiten dieses Schneidwerks nicht eingegangen wird. Das im Schneidwerk 44 in Partikel zerschnittene Abfallmaterial fällt in einen Abfallbehälter 43, der unterhalb des Schneidwerks angeordnet ist. Ebenso kann die Ionisiereinrichtung 49 statt vor dem UV-Strahler 16 unterhalb des Schneidwerks 44 angebracht sein.

Diese Ausführungsform der Vorrichtung 10 kommt im allgemeinen mit einem einzigen UV-Strahler 16 aus, wodurch weniger Wärme entsteht, die in jedem Fall abgeführt werden muß. Bei dieser Vorrichtung kann es sich u.U. auch als zweckmäßig erweisen, das Transportband 40 ausreichend lang zu gestalten, um so das Schneidwerk 44 und den Abfallbehälter 43 außerhalb des Raumes anzuordnen, in welchem das Abfallmaterial anfällt. Dadurch ist sichergestellt, daß Partikel bzw. Flitter aus zerschnittenem, fotovernetzbarem Abfallmaterial weitgehend von dem Verarbeitungsraum für das fotovernetzbare Material ferngehalten wird.

Jede der Ausführungsformen der Vorrichtung 10 ist mit Laufrollen 21 ausgerüstet und somit mobil.

## Patentansprüche

1. Vorrichtung zur Aufbereitung von Sondermüll aus fotovernetzbarem Abfallmaterial zu Hausmüll, bestehend aus einem mit einem Eingabetrichter (6; 41) ausgerüsteten Gehäuse (13), in dem ein oder mehrere UV-Strahler (1; 15; 16) nahe dem Transportweg des Abfallmaterials (17) angeordnet sind und das Abfallmaterial belichten und erwärmen und in dem ein Schneidwerk (9; 44) das Abfallmaterial durch Partikelschnitt zerkleinert.

2. Vorrichtung nach Anspruch 1, dadurch gekennzeichnet, daß das Gehäuse (13) als abnehm- oder abklappbarer Aufsatz auf einem, nach oben hin offenen, prismatischen Behälter (14) aufsitzt, der eine Tür (18) aufweist und einen herausnehmbaren Abfallsack (19) für das zerkleinerte Abfallmaterial enthält.

3. Vorrichtung nach Anspruch 1, dadurch gekennzeichnet, daß der Eingabetrichter (6) in einem Eingabeschlitz (20) eines Einsatzes (4) mündet, daß im Eingabeschlitz Einzugswalzen (3) angeordnet sind, die das Abfallmaterial (17) mittels Flügeln (22), die auf Transportwellen (2) aufsitzen, in das darunter liegende Schneidwerk (9) befördern und daß die Einzugswalzen (3), die Transportwellen (2) und das Schneidwerk (9) in dem Einsatz (4) aus UV-durchlässigem Glas oder Kunststoff angeordnet sind.

4. Vorrichtung nach Anspruch 3, dadurch gekennzeichnet, daß zu beiden Seiten und außerhalb des Einsatzes (4) zumindest je ein UV-Strahler (1) horizontal oder senkrecht in einem gekrümmten UV-Reflektor (11) angeordnet ist und jeder der UV-Strahler über eine Luftzuführung (5) mit Kühlluft beaufschlagt ist.

5. Vorrichtung nach Anspruch 3, dadurch gekennzeichnet, daß eine der Einzugswalzen (3) und das Schneidwerk (9) über eine endlos geschlossene Zahnradkette (23), die über Zahnräder (24, 25) auf der Achse der Einzugswalze (3) und einer Welle (26) des Schneidwerks (9) geführt ist, gemeinsam angetrieben sind und daß ein Motor (27) über eine weitere Zahnradkette (28) eine erste Schneidwelle (29) des Schneidwerks (9) in Bewegung versetzt, auf der ein Zahnrad (30) aufsitzt, das mit einem Zahnrad (31) auf einer zweiten Schneidwelle (32) des Schneidwerks (9) kämmt und daß dieses Zahnrad (31) mit einem Zahnrad (46) auf der Welle (26) des Schneidwerks (9) in Eingriff ist.

6. Vorrichtung nach Anspruch 2, dadurch gekennzeichnet, daß die UV-Strahler (1) durch einen Schalter, der beim Aufsitzen des Gehäuses (13) auf dem Behälter (14) betätigbar ist, einschaltbar sind.

7. Vorrichtung nach einem oder mehreren der Ansprüche 1 bis 6, dadurch gekennzeichnet, daß im Eingabeschlitz (20) eine Fotozelle (33) angeordnet ist, die das Ein- und Ausschalten des Motors (27) entsprechend dem Ein- und Durchlauf des Abfallmaterials (17) steuert.

8. Vorrichtung nach Anspruch 1, dadurch gekennzeichnet, daß in dem Gehäuse (13) das Schneidwerk (9) dem oder den UV-Strahlern (1) nachgeschaltet ist.

9. Vorrichtung nach Anspruch 1, dadurch gekennzeichnet, daß das Schneidwerk (9) dem oder den UV-Strahlern (15) vorgeschaltet ist.

10. Vorrichtung nach Anspruch 9, dadurch gekennzeichnet, daß unterhalb des Eingabeschlitzes (20) das Schneidwerk (9) angeordnet ist, von dem eine Schneidwelle (34) direkt von einem Motor (35) angetrieben ist, daß unterhalb des Schneidwerks ein zylindrischer Einsatz (36) aus UV-durchlässigem Glas oder Kunststoff in der Vorrichtung (10) angeordnet ist, die eine Klapptür (37) aufweist und daß an jeder der Innenseiten der Vorrichtung (10) zumindest ein stabförmiger UV-Strahler (15) senkrecht oder waagerecht angebracht ist.

11. Vorrichtung nach Anspruch 1, dadurch gekennzeichnet, daß in dem Gehäuse (13) ein horizontal über zwei Umlenkrollen (38, 39) endlos umlaufendes Transportband (40) angeordnet ist, daß der Eingabetrichter (41) sich in das Innere des Gehäuses (13) erstreckt und oberhalb des Transportbandes (40) endet, daß zumindest ein UV-Strahler (16) oberhalb des Transportbandes und im Abstand von dem Eingabetrichter angebracht ist und daß ein Schneidwerk (44) seitlich versetzt zu dem und unterhalb des Transportbandes vorhanden ist.

12. Vorrichtung nach Anspruch 11, dadurch gekennzeichnet, daß der Eingabetrichter (41) im Gehäuseinneren in Bewegungsrichtung des Obertrums des Transportbandes (40) gekrümmt ist.

13. Vorrichtung nach Anspruch 11, dadurch gekennzeichnet, daß ein Ableitelement (42) an dem Transportband (40) im Bereich der einen Umlenkrolle (39) anliegt und das Abfallmaterial (17) in das Schneidwerk (44) abführt, unterhalb von dem ein Abfallbehälter (43) angeordnet ist.

14. Vorrichtung nach einem oder mehreren der Ansprüche 1 bis 13, dadurch gekennzeichnet, daß eine Ionisierungseinrichtung (49) im Inneren des Gehäuses mit hochfrequenter Spannung beaufschlagt ist.
